# DEMANDE DE BREVET EUROPEEN

(11) **EP 2 905 035 A1**
(43) Date de publication de la demande: **12.08.2015**
(21) Numéro de dépôt: 15150377.8
(22) Date de dépôt: 07.01.2015
(51) Int. Cl.: A61L 9/04, A61L 9/12

(54) **Dispositif de diffusion d'une fragrance avec une multitude d'ouvertures**

(30) Priorité: 07.01.2014 FR 1450072
(71) Demandeur: Techniplast, 27400 Louviers (FR)
(72) Inventeur: LAMBOUX, Jean-Philippe, 27370 Saint Didier des Bois (FR); LEQUERE, Frédéric, 27190 Nogent le Sec (FR)
(74) Mandataire: Petit, Maxime

(57) **Abrégé**

L'invention concerne un dispositif de diffusion d'une fragrance qui comprend :
- deux parois (30, 32) en regard définissant entre elles une cavité (C) qui s'étend transversalement jusqu'à une paroi périphérique (40) pourvue d'une ouverture (42) pour la diffusion de fragrance à l'extérieur du dispositif,
- plusieurs ouvertures (O1, 02) aménagées dans chaque paroi et qui la traversent de manière à permettre, pour une première paroi (30), le passage de la fragrance dans la cavité à partir d'une première zone (Z1) qui est adjacente à la première paroi (30) et séparée de la cavité par cette paroi et, pour la deuxième paroi (32), le passage de la fragrance de la cavité à une deuxième zone (Z2) qui est adjacente à la deuxième paroi (32) et séparée de la cavité par cette deuxième paroi,
- un conduit (44,46) traversant chacune des deux parois et s'étendant dans la zone adjacente à la paroi concernée afin de mettre en communication chaque zone avec la cavité.

## Description

L'invention concerne un dispositif de diffusion d'une fragrance telle qu'un parfum.

On connait du document FR 2 958 854 un diffuseur de fragrance comprenant :
- deux bouchons verseurs montés tête-bêche et présentant chacun un tube d'écoulement,
- une plaquette de matière poreuse montée entre les deux bouchons verseurs,
- une bague extérieure pourvue, en regard de la plaquette, d'au moins un orifice de diffusion de la fragrance.

Chaque bouchon verseur comprend également un trou d'écoulement qui permet le passage de la fragrance dans la plaquette de matière poreuse, puis vers l'extérieur du dispositif. La fragrance est par exemple contenue dans un flacon disposé au dessus du diffuseur.

Bien que satisfaisant ce diffuseur pourrait toutefois être simplifié.

A cet effet, l'invention vise un dispositif de diffusion d'une fragrance, caractérisé en ce qu'il comprend :
- deux parois en regard espacées l'une de l'autre suivant un axe longitudinal L vertical de manière à définir entre elles une cavité C qui s'étend transversalement jusqu'à une paroi périphérique pourvue d'au moins une ouverture pour la diffusion de fragrance à l'extérieur du dispositif, ladite au moins une ouverture mettant en communication la cavité avec l'extérieur du dispositif,
- plusieurs ouvertures aménagées dans chacune des deux parois en regard et qui traversent la paroi concernée de manière à permettre, pour une première des deux parois située au-dessus de la deuxième paroi, le passage de la fragrance liquide d'une première zone Z1 dans la cavité en vue de la diffusion de ladite fragrance à l'extérieur du dispositif via ladite au moins une ouverture de diffusion, la première zone étant adjacente à ladite première paroi, au-dessus de celle-ci et séparée de la cavité par cette première paroi et, pour la deuxième des deux parois, le passage de la fragrance liquide de la cavité à une deuxième zone Z2 qui est adjacente à ladite deuxième paroi, en dessous de celle-ci et séparée de la cavité par cette deuxième paroi,
- au moins un conduit qui traverse chacune des deux parois en regard et s'étend dans la zone adjacente à la paroi concernée de manière à mettre en communication chaque zone adjacente à chaque paroi avec la cavité, ledit au moins un conduit aménagé dans la première paroi étant un conduit de reprise d'air qui reprend directement de l'air de l'extérieur du dispositif via ladite au moins une ouverture et la cavité, tandis que ledit au moins un conduit aménagé dans la deuxième paroi est un conduit d'écoulement de la fragrance liquide provenant de la cavité.

Dans le dispositif selon un mode de réalisation de l'invention, la fragrance (ex : parfum) passe à travers les ouvertures de la première paroi (paroi supérieure lorsque le dispositif est en position verticale de fonctionnement, la fragrance liquide étant contenue dans la première zone, notamment d'un flacon supérieur retourné) et, lorsque la cavité est libre de tout obstacle, chute dans la cavité (espace de diffusion) en produisant un goutte à goutte. Les gouttes tombent par gravité sur la deuxième paroi en regard (paroi inférieure également appelée collecteur) et s'écoulent par ledit au moins un conduit relié à cette paroi dans la deuxième zone adjacente à celle-ci (notamment dans un flacon inférieur).

Cette configuration du dispositif assure ainsi, avec les mêmes moyens, à la fois la diffusion de fragrance dans la cavité et à l'extérieur et la collecte des gouttes pour produire le goutte à goutte en dessous du dispositif (notamment dans un flacon inférieur). Le dispositif selon l'invention est donc plus simple que celui de l'art antérieur dans lequel les fonctions de diffusion et de goutte à goutte sont dissociées. Il n'est pas non plus prévu de matériau poreux à installer dans la cavité, ce qui simplifie le dispositif et le rend moins onéreux.

On notera que le dispositif selon un mode de réalisation de l'invention peut comporter une cavité dépourvue de tout obstacle ou non entre les deux parois en regard.

La diffusion de fragrance a lieu dans la cavité, puis à l'extérieur du dispositif et la fragrance liquide non diffusée dans la cavité passe de la cavité audit au moins un conduit d'écoulement. La fragrance liquide ne passe donc pas directement de la première zone à la deuxième zone comme dans l'art antérieur.

Par ailleurs, dans l'art antérieur précité, pour assurer le goutte à goutte, de très petits trous (ex : 0,2 à 0,5 mm) sont nécessaires et ceux-ci ont tendance à se boucher en présence des produits gras qui rentrent dans la composition des fragrances (parfums). Avec le dispositif selon l'invention, la multitude d'ouvertures dans les parois en regard limite ce phénomène.

Contrairement au dispositif de l'art antérieur précité, l'air de reprise qui compense le volume de fragrance s'écoulant à travers les ouvertures de la première paroi vient de l'extérieur du dispositif et passe à travers ladite au moins une ouverture de diffusion de fragrance, puis dans la cavité et dans ledit au moins un conduit de reprise d'air. Ceci permet de dissocier la compensation d'air dans le flacon qui dispense sa fragrance de l'écoulement de cette fragrance et, donc, du goutte à goutte. Dans l'art antérieur, les flacons communiquent entre eux par des tubes d'écoulement et de passage d'air. Ainsi, la pression ou la dépression de l'un ou l'autre des flacons influe sur le fonctionnement du goutte à goutte (par exemple, lorsqu'un équilibre de pressions doit s'établir), ce qui n'est pas le cas du dispositif selon l'invention.

On notera que ledit au moins un conduit traversant chaque paroi fait exclusivement saillie à l'intérieur de la zone adjacente concernée et non à l'intérieur de la cavité entre les parois afin de ne pas gêner l'écoulement du liquide dans le ou les conduits d'écoulement de la deuxième paroi.

Selon d'autres caractéristiques possibles, prises isolément ou en combinaison l'une avec l'autre :
- ledit au moins un conduit de reprise d'air s'étend dans la première zone Z1 sur une hauteur adaptée pour que l'extrémité débouchante dudit au moins un conduit de reprise d'air soit à une cote supérieure à celle du niveau de fragrance liquide contenue dans la première zone Z1 ; cet agencement garantit la fonction de reprise de l'air extérieur au dispositif ;
- les ouvertures d'une des deux parois en regard sont décalées géométriquement par rapport aux ouvertures de l'autre paroi; le fait que les ouvertures d'une paroi ne soient pas en vis-à-vis des ouvertures de la paroi en regard permet d'éviter que les gouttes issues des ouvertures supérieures ne tombent sur les ouvertures inférieures, ce qui risquerait de les boucher ;

- au moins certaines ouverture(s) aménagée(s) dans les deux parois en regard sont prolongées chacune par un col qui fait saillie dans la cavité par rapport à la paroi concernée ; cet agencement permet d'éviter que des gouttes n'obstruent ces ouvertures de la deuxième paroi lorsqu'elles tombent sur celle-ci et s'écoulent dessus ; ces ouvertures de la deuxième paroi autorisent l'évacuation d'air présent dans le flacon inférieur pendant que la fragrance liquide non diffusée s'écoule dans ledit flacon ;
- le dispositif comprend plusieurs éléments retardateur d'écoulement de la fragrance liquide comprenant chacun un corps ou obstacle de forme générale allongée, les corps ou obstacles s'étendant dans la cavité de manière à relier chacun deux ouvertures respectives des deux parois en regard, permettant ainsi à la fragrance liquide d'être acheminée via chaque élément retardateur d'écoulement d'une ouverture de la première paroi à une ouverture de la deuxième paroi ; ces corps ou obstacles retardent l'écoulement en le déviant de son trajet direct, notamment vertical, de la première à la deuxième paroi ; ces corps ou obstacles offrent par exemple plusieurs chemins simultanés possibles pour l'écoulement au lieu d'un seul (trajet direct) ;
- les corps des éléments retardateur d'écoulement de la fragrance sont formés de plusieurs tubes qui s'étendent dans la cavité de manière à ce que chaque tube relie entre elles deux ouvertures respectives des deux parois en regard, permettant ainsi à la fragrance liquide d'être acheminée via chaque tube d'une ouverture de la première paroi à une ouverture de la deuxième paroi ; dans cette configuration, les tubes retardent l'écoulement des gouttes de fragrance, prolongeant ainsi leur séjour dans la cavité et donc le phénomène de diffusion ;
- chaque tube comprend, agencés suivant sa longueur, au moins deux compartiments internes séparés l'un de l'autre par une cloison transversale, ledit tube étant percé suivant sa longueur d'une pluralité d'orifices latéraux qui permettent à la fragrance liquide présente dans un compartiment agencé à proximité d'une première paroi d'en sortir, de s'écouler le long de la face externe dudit tube jusqu'à atteindre la deuxième paroi et ensuite ledit au moins un conduit aménagé dans ladite deuxième paroi. Cet agencement créé en quelque sorte des chicanes pour l'écoulement de la fragrance liquide en empêchant un passage direct vertical d'une ouverture à l'autre ; le temps de présence de la fragrance dans la cavité (notamment par stagnation et écoulement) est ainsi augmenté, ce qui permet de prolonger l'effet de diffusion et donc de l'améliorer ;
- les éléments retardateur d'écoulement de la fragrance s'étendent dans la cavité suivant l'axe longitudinal ;
- le dispositif comprend, de part et d'autre des deux parois, dans chaque zone adjacente à chaque paroi, des moyens de fixation destinés chacun à fixer au dispositif un col d'un flacon ;
- chaque paroi présente une forme générale concave dont la concavité est orientée vers la cavité et comporte une région centrale ;
- ledit au moins un conduit est situé dans la région centrale ;
- les éléments retardateur d'écoulement de la fragrance sont disposés autour dudit au moins un conduit ;
- le dispositif comprend un ou plusieurs éléments en matière absorbante aménagés sur chacune des deux parois en regard ; ce ou ces éléments sont aptes à absorber le liquide qui tombe dessus en provenance du flacon supérieur pour y retenir de manière temporaire le liquide, évitant ainsi les risques de coulure lors du retournement du dispositif.

Selon un deuxième aspect, l'invention vise également un système de diffusion d'une fragrance, caractérisé en ce qu'il comprend :
- un dispositif de diffusion tel que brièvement exposé ci-dessus,
- un premier flacon supérieur ayant un premier col dont l'ouverture est disposée en vis-à-vis de la première des deux parois en regard dite paroi supérieure, un second flacon inférieur ayant un deuxième col dont l'ouverture est disposée en vis-à-vis de la deuxième paroi dite inférieure, le premier flacon supérieur comprenant la fragrance liquide qui, par écoulement sous forme de goutte à goutte à travers les ouvertures, provoque, d'une part, la diffusion de la fragrance dans la cavité et à l'extérieur du dispositif *via* ladite au moins une ouverture et, d'autre part, le goutte à goutte dans le second flacon inférieur.

Ce système présente les mêmes avantages et caractéristiques que ceux mentionnés en relation avec le dispositif de diffusion mentionné plus haut et ils ne seront donc pas répétés ici.

Selon un troisième aspect, l'invention vise un procédé de diffusion d'une fragrance dans un dispositif de diffusion d'une fragrance, caractérisé en ce que le dispositif comprend :
- deux parois en regard espacées l'une de l'autre suivant un axe longitudinal L vertical de manière à définir entre elles une cavité C qui s'étend transversalement jusqu'à une paroi périphérique pourvue d'au moins une ouverture pour la diffusion de fragrance à l'extérieur du dispositif, ladite au moins une ouverture mettant en communication la cavité avec l'extérieur du dispositif,
- plusieurs ouvertures aménagées dans chacune des deux parois en regard et qui traversent la paroi concernée de manière à permettre, pour une première des deux parois située au-dessus de la deuxième paroi, le passage de la fragrance liquide d'une première zone Z1 dans la cavité en vue de la diffusion de ladite fragrance à l'extérieur du dispositif via ladite au moins une ouverture de diffusion, la première zone étant adjacente à ladite première paroi au-dessus de celle-ci et séparée de la cavité par cette première paroi et, pour la deuxième des deux parois, le passage de la fragrance liquide de la cavité à une deuxième zone Z2 qui est adjacente à ladite deuxième paroi, en dessous de celle-ci et séparée de la cavité par cette deuxième paroi,
- au moins un conduit qui traverse chacune des deux parois en regard et s'étend dans la zone adjacente à la paroi concernée de manière à mettre en communication chaque zone adjacente à chaque paroi avec la cavité, ledit au moins un conduit aménagé dans la première paroi étant un conduit de reprise d'air, tandis que ledit au moins un conduit aménagé dans la deuxième paroi est un conduit d'écoulement de la fragrance provenant de la cavité, selon le procédé la fragrance liquide contenue dans la première zone Z1 s'écoule à travers les ouvertures de la première paroi pour que, d'une part, la fragrance diffuse dans la cavité C et à l'extérieur du dispositif via ladite au moins une ouverture et, d'autre part, la partie restante de la fragrance liquide non diffusée s'écoule dans la deuxième zone Z2 par ledit au moins un conduit, l'écoulement de fragrance liquide à travers les ouvertures de la première paroi étant permis par la reprise d'air extérieur via ladite au moins une ouverture, la cavité C et ledit au moins un conduit de reprise d'air.

Selon d'autres caractéristiques possibles du procédé :
- l'écoulement de fragrance liquide depuis la première zone à travers les ouvertures de la première paroi est dévié, lors de son passage dans la cavité, d'un trajet direct pour atteindre la deuxième paroi afin d'augmenter le temps de présence dudit écoulement dans ladite cavité ; la déviation de l'écoulement a un effet retardateur sur l'écoulement ; l'augmentation du temps de présence dans la cavité a pour effet de prolonger, et donc d'améliorer, l'effet de diffusion de la fragrance à l'extérieur du dispositif ;
- l'écoulement de fragrance liquide est dévié par sa rencontre avec une pluralité d'obstacles présents dans la cavité C ; l'écoulement longe ainsi ou passe à travers les obstacles ou corps s'étendant dans la cavité et qui dévient ledit écoulement d'un trajet qui serait plus direct en l'absence de tels obstacles ou corps ;
- la diffusion de fragrance a lieu dans un système de diffusion d'une fragrance, comprenant :
- un dispositif de diffusion de fragrance,
- un premier flacon supérieur ayant un premier col dont l'ouverture est disposée en vis-à-vis de la première des deux parois en regard dite paroi supérieure, un second flacon inférieur ayant un deuxième col dont l'ouverture est disposée en vis-à-vis de la deuxième paroi dite inférieure, le premier flacon supérieur comprenant la fragrance liquide. La fragrance, par écoulement sous forme de goutte à goutte à travers les ouvertures provoque, d'une part, la diffusion de la fragrance dans la cavité C et à l'extérieur du dispositif *via* ladite au moins une ouverture et, d'autre part, le goutte à goutte dans le second flacon inférieur.

D'autres caractéristiques et avantages apparaîtront au cours de la description qui va suivre, donnée uniquement à titre d'exemple non limitatif et faite en référence aux dessins annexés, sur lesquels :
- la figure 1 est une vue générale schématique en coupe axiale d'un dispositif de diffusion d'une fragrance selon un premier mode de réalisation de l'invention ;
- la figure 2 est une vue de dessous du dispositif de la figure 1 retourné avec une autre répartition d'ouvertures dans la paroi 30;
- la figure 3 est une vue générale schématique en coupe axiale d'un dispositif de diffusion d'une fragrance selon un deuxième mode de réalisation de l'invention ;
- la figure 4 est une vue schématique en coupe axiale d'un dispositif de diffusion d'une fragrance qui intègre de manière artificielle plusieurs variantes de réalisation du dispositif de la figure 3 ;
- la figure 5 est une vue schématique en coupe axiale d'un dispositif de diffusion d'une fragrance selon une variante de réalisation supplémentaire du dispositif de la figure 1 ;
- la figure 6 est une vue générale schématique d'un système de diffusion d'une fragrance selon un mode de réalisation de l'invention.

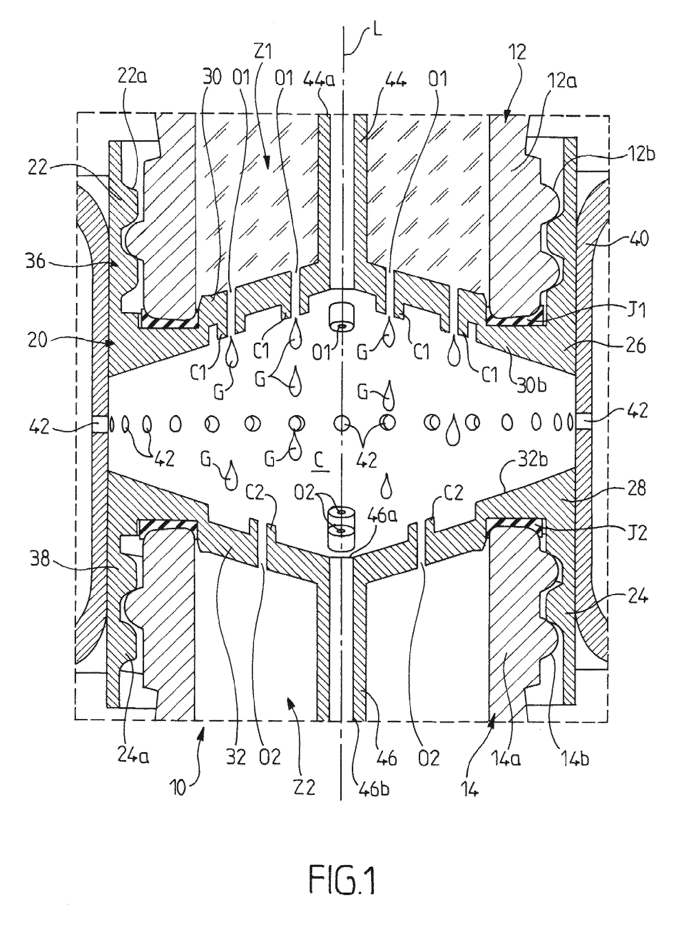

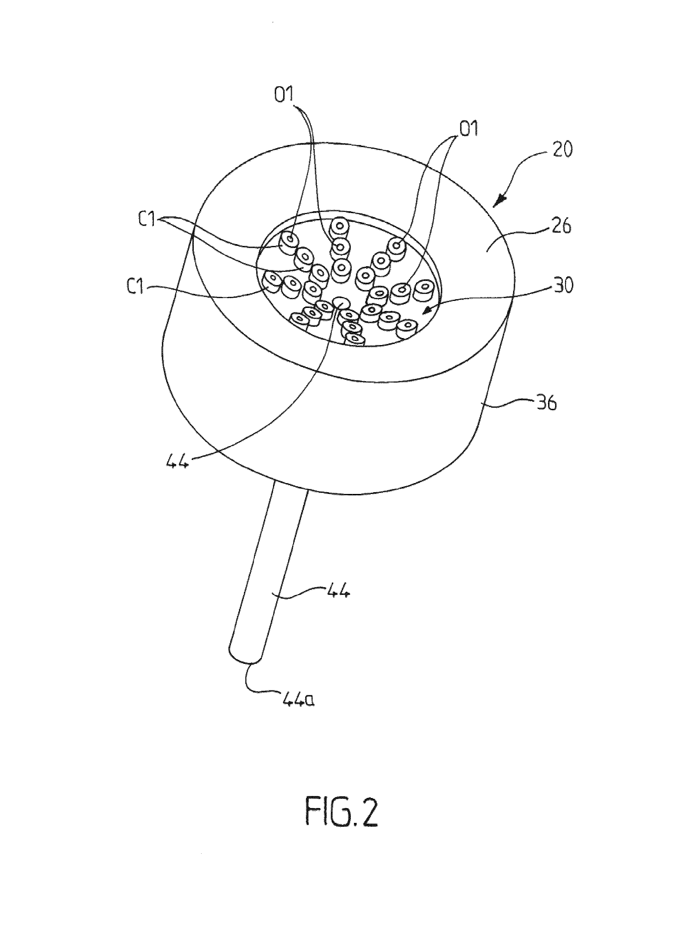

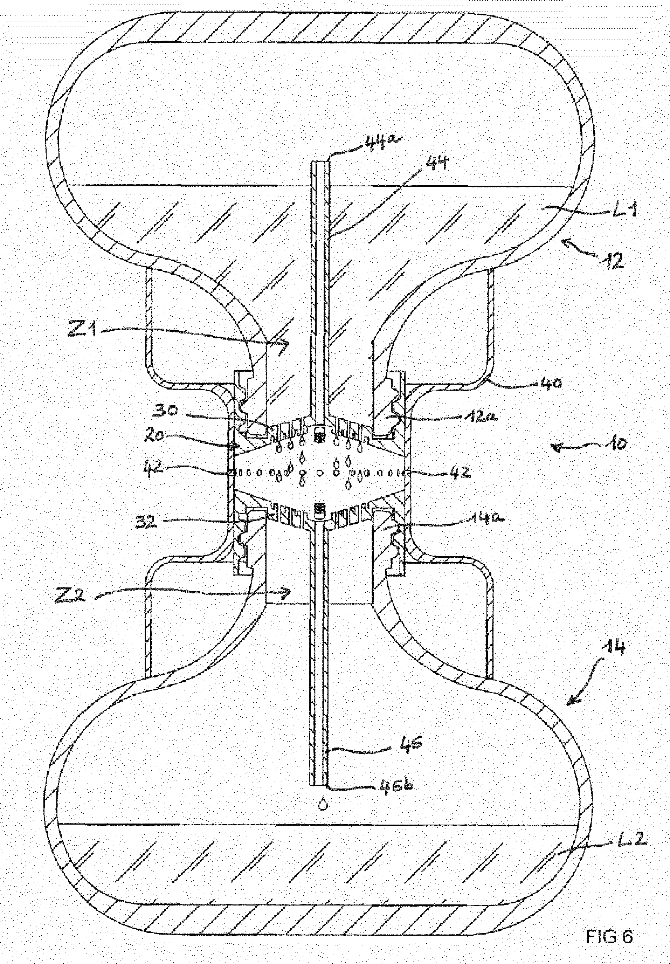

Comme représenté partiellement à la figure 1 et désigné par la référence générale notée 10, un système de diffusion d'une fragrance selon un mode de réalisation de l'invention comprend un dispositif de diffusion d'une fragrance 20 et deux flacons 12, 14 montés tête bêche dont seuls les cols respectifs 12a, 14a sont représentés. Le système 10 est représenté dans sa totalité à la figure 6 avec la configuration de la figure 2. Toutefois, la description qui va suivre des figures 1, 2 et 6 ne dépend pas d'une configuration particulière telle que celle de la figure 2.

Un premier flacon supérieur 12 contenant la fragrance (ex : un parfum d'ambiance) à diffuser est monté en position retournée au-dessus du dispositif 20 avec son col 12a orienté vers le bas.

Le dispositif 20 comprend des premiers moyens de fixation 22 qui sont destinés à fixer le col 12a audit dispositif. Ces moyens de fixation sont par exemple réalisés sous la forme d'un filetage interne 22a pour le vissage avec le filetage externe 12b sur la surface externe du col 12a.

Un deuxième flacon inférieur 14 destiné à récupérer la fragrance non diffusée provenant du flacon supérieur 12 est monté sous le dispositif 20 avec son col 14a orienté vers le haut.

Le dispositif de diffusion 20 comprend des deuxièmes moyens de fixation 24 qui sont destinés à fixer le col 14a audit dispositif. Ces moyens de fixation sont par exemple réalisés sous la forme d'un filetage interne 24a pour le vissage avec le filetage externe 14b sur la surface externe du col 14a. D'autres moyens de fixation tels que par encliquetage peuvent être envisagés.

Le système 10 et son dispositif 20 ainsi que les deux flacons sont agencés verticalement sur la figure 1 afin que la fragrance contenue dans le flacon supérieur 12 puisse s'écouler par gravité.

Le dispositif 20 comprend deux pièces, à savoir une pièce supérieure 26 et une pièce inférieure 28 disposées en regard et espacées l'une de l'autre suivant un axe longitudinal L qui, ici, est orienté verticalement.

Les deux pièces 26, 28 comprennent chacune une paroi 30, 32 disposée en vis-à-vis l'une de l'autre et qui définissent entre elles, suivant l'axe longitudinal L, une cavité centrale C.

Les parois 30, 32 s'étendent transversalement à partir de leur région centrale vers leur périphérie au niveau de laquelle s'étend axialement une paroi 36, 38. Les deux parois d'extension axiale 36, 38 des pièces 26, 28 s'étendent dans des directions opposées et en éloignement de la cavité C. Les moyens de fixation 22, 24 sont aménagés sur la surface intérieure de ces parois. On notera que le col 12a, 14a de chaque flacon, une fois fixé au dispositif 20, vient en butée contre un joint respectif J1, J2 aménagé dans un évidement de la face externe 30a, 32a de la paroi d'extension transversale considérée.

Comme représenté, chaque paroi transversale 30, 32 présente une forme générale concave dont la concavité est orientée vers la cavité C. Chaque paroi a plus particulièrement une forme d'entonnoir convergeant vers la région centrale.

Chaque pièce 26, 28 présente de façon générale une symétrie de révolution axiale à quelques détails de réalisation près. Ainsi, les parois d'extension axiale 36, 38 ont chacune une forme sensiblement cylindrique leur conférant une forme de jupe.

Le dispositif 20 comprend également à sa périphérie une paroi périphérique 40 ayant une forme générale de bague entourant l'ensemble formé des deux pièces 26, 28 en vis-à-vis et de la cavité centrale séparatrice C. Cette paroi périphérique 40 forme une pièce d'habillage et est fixée sur chacun des ensembles précités par exemple par collage, soudage ou par un autre moyen approprié.

La paroi périphérique 40 est munie d'au moins une ouverture 42 pour la diffusion de fragrance à l'extérieur du dispositif. Ladite au moins une ouverture 42 est pratiquée dans la région de la paroi qui est située autour de la cavité C de manière à mettre en communication cette cavité avec l'extérieur du dispositif. En pratique, pour une meilleure répartition de la diffusion de la fragrance, plusieurs ouvertures 42 sont disposées sur le pourtour de la paroi périphérique 40 comme représenté sur la figure 1, dans une région équatoriale du dispositif.

Chacune des parois d'extension transversale 30, 32 comporte une pluralité d'ouvertures traversantes respectivement notées O1, 02 qui mettent chacune en communication, avec la cavité C, une zone externe au dispositif, notée respectivement Z1, Z2, et adjacente à la paroi concernée 30, 32. Ainsi que représenté sur la figure 1, chaque zone externe Z1, Z2 est une zone interne au col d'un flacon.

La figure 2 représente un autre exemple possible de répartition des ouvertures O1 dans la paroi 30. Les ouvertures sont disposées en séries qui regroupent chacune plusieurs ouvertures, les séries étant agencées suivant une disposition radiale. D'autres configurations alternatives peuvent être utilisées (ex : disposition en couronnes concentriques..).

Les ouvertures disposées dans la paroi supérieure (ici les ouvertures O1 dans la paroi 30) sont dimensionnées pour permettre leur traversée par des gouttes de fragrance G provenant de la zone Z1 et leur chute à l'intérieur de la cavité comme illustré sur la figure 1. La multitude d'ouvertures perforant la paroi assure une capacité de diffusion donnée. Ce paramètre dépend des applications envisagées.

On notera que chaque ouverture O1, 02 est prolongée du côté de la face interne 30b, 32b de la paroi 30, 32 par un col respectivement noté c1, c2 et qui fait saillie dans la cavité par rapport à la paroi concernée. Cette configuration permet d'éviter que les ouvertures ne se bouchent lorsque les gouttes de fragrance tombent sur la paroi 32 qui fait office de collecteur (ou sur la paroi 30 lorsque celle-ci est à son tour en dessous et fait alors office de collecteur).

Les ouvertures O1 et 02 des deux parois 30, 32 en regard ne sont pas en vis-à-vis l'une de l'autre mais décalées transversalement pour réduire le risque de bouchage en cas de chute des gouttes sur les ouvertures inférieures.

Chacune des parois d'extension transversale 30, 32 comporte également un conduit noté respectivement 44, 46, aménagé dans la région centrale de la paroi (au fond de la concavité) et traversant cette dernière de manière à mettre en communication la zone adjacente concernée (Z1, Z2) avec la cavité C. Plus particulièrement, chaque conduit 44, 46 s'étend longitudinalement (ici verticalement) dans la zone adjacente concernée à la façon d'une cheminée. Le conduit supérieur 44 s'étend à l'intérieur du flacon supérieur 12 sur une hauteur suffisante pour que l'extrémité débouchante 44a du conduit supérieur 44 soit toujours au-dessus du niveau de la fragrance liquide L1 contenue dans la zone Z1 (figure 6). Il en est de même avec le conduit 46 lorsque le dispositif est renversé et qu'il devient un conduit supérieur. Sur les figures 1 et 3 à 5, l'extrémité débouchante du conduit 44 n'a pas été prolongée comme sur la figure 6 par souci de simplification.

Chaque conduit débouche, à son extrémité qui est opposée à l'extrémité libre débouchante, au niveau de la face interne 30b, 32b de la paroi concernée sans toutefois faire saillie dans la cavité. Les ouvertures O1 et O2 entourent donc respectivement les conduits 44 et 46.

Le conduit supérieur 44 fait office de conduit de reprise d'air qui reprend directement de l'air de l'extérieur du dispositif via les ouvertures 42 et non du flacon inférieur. Cet agencement est plus efficace car il est moins sensible aux variations de pression d'un flacon à l'autre. Ces variations de pression peuvent être, par exemple, générées lorsque le flacon du haut est exposé au soleil derrière une fenêtre et celui du bas à l'ombre, ou le contraire, ou bien lorsque le flacon du bas est posé sur un radiateur alors que celui du haut est exposé à un courant d'air froid. Les changements de températures influent sur les pressions par l'effet de dilatation des bases alcooliques qui sont présente dans les parfums. Dans le dispositif selon l'invention, l'air de reprise est en pression stable puisqu'il provient de l'extérieur. On notera que le conduit est dimensionné (notamment par son diamètre) pour permettre un goutte à goutte de fragrance dans la cavité qui est adapté au nombre d'ouvertures de la paroi et à leur diamètre et qui est satisfaisant en termes de puissance de diffusion (capacité à diffuser des molécules odorantes dans l'atmosphère). En effet, la vitesse d'écoulement et donc du goutte à goutte dépend notamment de la capacité du dispositif à reprendre de l'air.

Le conduit inférieur 46 récupère les gouttes tombées sur la paroi inclinée 32 et acheminées par gravité jusqu'audit conduit. Le conduit 46 (pénètre dans le flacon inférieur 14) assure ainsi l'écoulement de fragrance dans la zone Z2 et dans le flacon inférieur de la fragrance qui n'a pas été diffusée.

Lors du fonctionnement du système 10 de diffusion d'une fragrance, le liquide (fragrance) présent dans la zone Z1 du flacon supérieur 12 retourné traverse les ouvertures O1, puis forme des gouttes G en s'échappant desdites ouvertures. Les gouttes chutent par gravité à l'intérieur de la cavité sur toute la hauteur de celle-ci et s'éclatent en atteignant la paroi inférieure 32 du collecteur. La diffusion de la fragrance s'opère ainsi durant la chute des gouttes mais principalement lors de l'éclatement des gouttes sur la paroi 32. La diffusion s'opère radialement ou transversalement dans l'air qui est présent à l'intérieur de la cavité C (zone de diffusion), puis à travers les ouvertures 42 pour sortir du dispositif 20.

Le goutte à goutte ainsi formé dans la cavité crée en quelque sorte une aspiration du liquide du flacon 12 et donc une dépression dans ledit flacon (effet de pompage). Cette dépression est compensée par l'air externe qui entre dans le flacon par le conduit de reprise d'air 44.

Tout le liquide s'écoulant sous forme de gouttes dans la cavité n'est pas diffusé dans cette dernière. En effet, une partie des gouttes qui atteignent la paroi inférieure 32 (agencée en pente en direction de l'entrée 46a du conduit d'écoulement 46) sont collectées sur cette paroi sans être diffusées. Ces gouttes s'écoulent le long de la paroi jusqu'au conduit 46, y pénètrent et s'écoulent à l'intérieur, puis dans le flacon inférieur 14, sous la forme d'un goutte à goutte. Lorsque le liquide « non diffusé » est passé dans le flacon inférieur le phénomène de diffusion prend fin. La contenance du flacon inférieur est dimensionnée pour que le niveau du liquide L2 transféré dans le flacon 14 soit situé en-dessous de l'extrémité inférieure libre 46b du conduit (figure 6).

Lorsqu'il n'y a plus de liquide dans le flacon supérieur 12 et que le flacon inférieur 14 est rempli (au moins partiellement), le système est retourné : Le flacon inférieur 14 devient le flacon supérieur et inversement, et le mode opératoire décrit ci-dessus se répète.

On notera que la vitesse d'écoulement du liquide et donc du goutte à goutte peut dépendre :
- du calibrage des trous 01,
- de la viscosité du parfum,
- du calibrage de la reprise d'air 44.

Les parois collectrices 30 et 32 sont inclinées en direction de leur région centrale (fond de la concavité) afin de pouvoir collecter le liquide dans le conduit central 46. Ceci permet également que les ouvertures 02 présentes dans la paroi 32 (le même raisonnement s'applique avec les ouvertures O1 présentes dans la paroi 30 quand le système est retourné) soient disposées à une altitude supérieure à celle d'un point bas de la paroi entourant le conduit 46 et autour duquel du liquide pourrait s'accumuler. Un tel agencement réduit considérablement le risque que du liquide collecté par la paroi 32 ne vienne obstruer les ouvertures 02 lors du fonctionnement du système.

Selon une variante non représentée, plusieurs conduits de reprise d'air 44 (resp. conduits d'écoulement 46) peuvent être aménagés sur la paroi 30 (resp. 32).

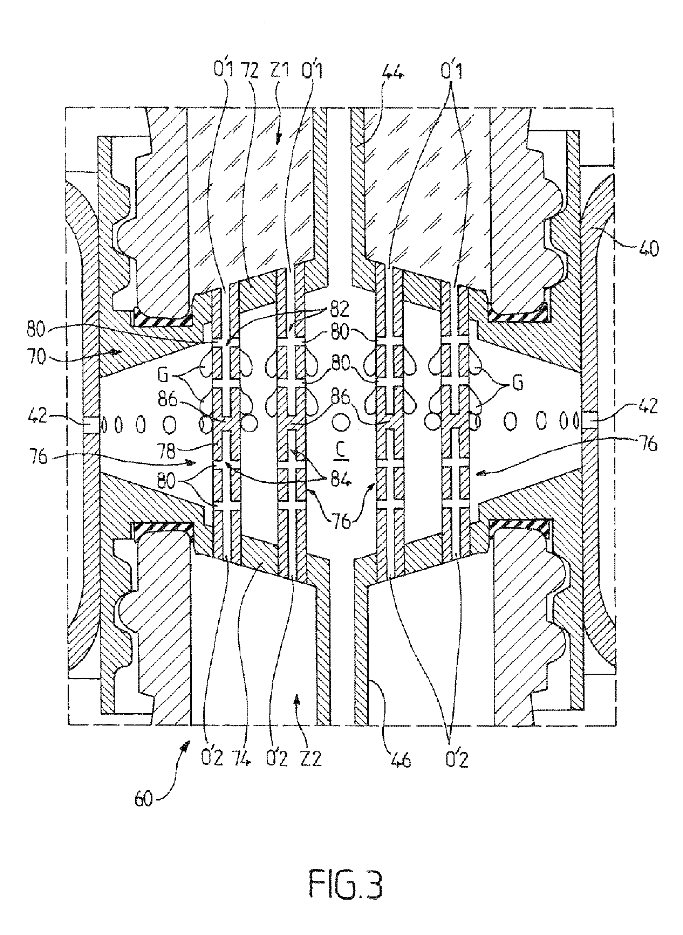

La figure 3 représente un système de diffusion d'une fragrance 60 selon un deuxième mode de réalisation de l'invention. Ce système 60 comprend un dispositif de diffusion d'une fragrance 70 qui diffère principalement du dispositif 20 de la figure 1 par la présence d'obstacles ou de corps déviant l'écoulement de fragrance liquide à l'intérieur de la cavité C tels que des tubes.

Les éléments identiques par rapport aux figures 1 et 2 conserveront les mêmes références et ne seront pas à nouveau décrits.

Les parois concaves supérieure 72 et inférieure 74, inclinées en direction de l'entrée de chaque conduit respectif 44, 46, sont également pourvues d'ouvertures O'1, O'2 traversant la paroi concernée dans son épaisseur, de manière à permettre la formation et le passage de gouttes de la zone adjacente Z1, Z2 à la cavité.

Plusieurs tubes 76 s'étendent longitudinalement à l'intérieur de la cavité, chacun entre deux ouvertures O'1 et O'2 qui sont situées en vis-à-vis l'une de l'autre et qui sont reliées par un tube. Chaque tube est par exemple inséré par l'intermédiaire de ses deux extrémités opposées respectivement dans les deux ouvertures O'1 et O'2 en correspondance. Alternativement, les tubes peuvent être fixés à la paroi concernée sans être introduits dans les ouvertures.

Chaque tube 76 est creux et comporte une paroi cylindrique 78 qui est percée d'une pluralité d'orifices latéraux 80 qui mettent en communication l'intérieur du tube et la cavité.

Plus particulièrement, le tube 76 est séparé en deux compartiments ou chambres allongés 82, 84, disposés l'un au-dessus de l'autre et séparés l'un de l'autre par une cloison transversale 86, par exemple à mi-hauteur du tube. Le premier compartiment 82 communique avec l'ouverture O'1 et le deuxième compartiment 84 communique avec l'ouverture O'2.

Les orifices latéraux 80 qui sont situés le plus bas dans le premier compartiment 82 sont disposés de préférence à distance de la cloison séparatrice 86 afin de ménager entre eux un espace qui peut se remplir de liquide (volume tampon).

En fonctionnement, le liquide s'écoule à travers les ouvertures O'1 et pénètre dans les premiers compartiments 82 de chaque tube. Le liquide s'accumule par gravité dans l'espace précité jusqu'à atteindre les orifices 80 les plus bas et sortir des tubes par ces derniers. Si le volume de liquide est plus grand, le compartiment 82 se remplit davantage jusqu'à atteindre les orifices supérieurs 80et à sortir par ces derniers. On notera que la section des orifices latéraux 80 et la section interne des compartiments 82, 84 est dimensionnée de manière adaptée au but poursuivi. Ainsi, par exemple, si l'on choisit de toutes petites sections pour les orifices latéraux 80, le liquide pourra monter dans le compartiment 82 de plus grande section et le remplir jusqu'en haut. Dans ce cas, c'est la section des orifices latéraux 80 qui définit la vitesse d'écoulement du parfum dans la cavité. Les gouttes de liquide sortant des tubes s'écoulent le long de leur face externe et, pour certaines, diffusent dans la cavité lors de cet écoulement. D'autres gouttes qui n'ont pas diffusé longent la face externe des tubes 76, atteignent la paroi inférieure 74 en pente (collecteur), glissent le long de celle-ci en direction du conduit central 46 et le traversent pour tomber dans le flacon inférieur.

Les tubes 76 ainsi que la paroi inférieure 74 jouent le rôle de collecteurs de liquide.

Par ailleurs, les tubes 76 forment des chicanes pour l'écoulement de liquide provenant des ouvertures O'1, ralentissant ainsi cet écoulement et donc le phénomène du goutte à goutte. Il s'ensuit que le temps de passage (écoulement) des gouttes à l'intérieur de la cavité est augmenté, ce qui permet d'augmenter le temps de diffusion et la capacité de diffusion du dispositif La gestion du temps de diffusion peut être contrôlée en sélectionnant de manière adaptée le nombre de tubes 76 et leur section transversale. L'agencement à deux compartiments décrit ci-dessus est facilement nettoyable dans la mesure où les deux compartiments sont accessibles par leurs extrémités respectives ouvertes.

Selon des variantes possibles non représentées :
- la hauteur des compartiments peut être différente d'un compartiment à l'autre, la cloison séparatrice étant alors agencée différemment ;
- l'agencement des orifices peut être différent, par exemple en quinconce ;
- le nombre des tubes et leur répartition dans la cavité peuvent être différents mais toujours adaptés au nombre et à l'agencement des ouvertures dans les parois en regard du dispositif ;
- plus de deux compartiments peuvent être agencés l'un au dessus-de l'autre afin d'augmenter l'effet de chicanes sur l'écoulement.

On notera que d'autres obstacles ou corps de déviation d'écoulement tels que des moyens formant des chicanes pour dévier et donc retarder l'écoulement peuvent alternativement être agencés dans la cavité en relation ou non avec des tubes tels que les tubes de goutte à goutte 76.

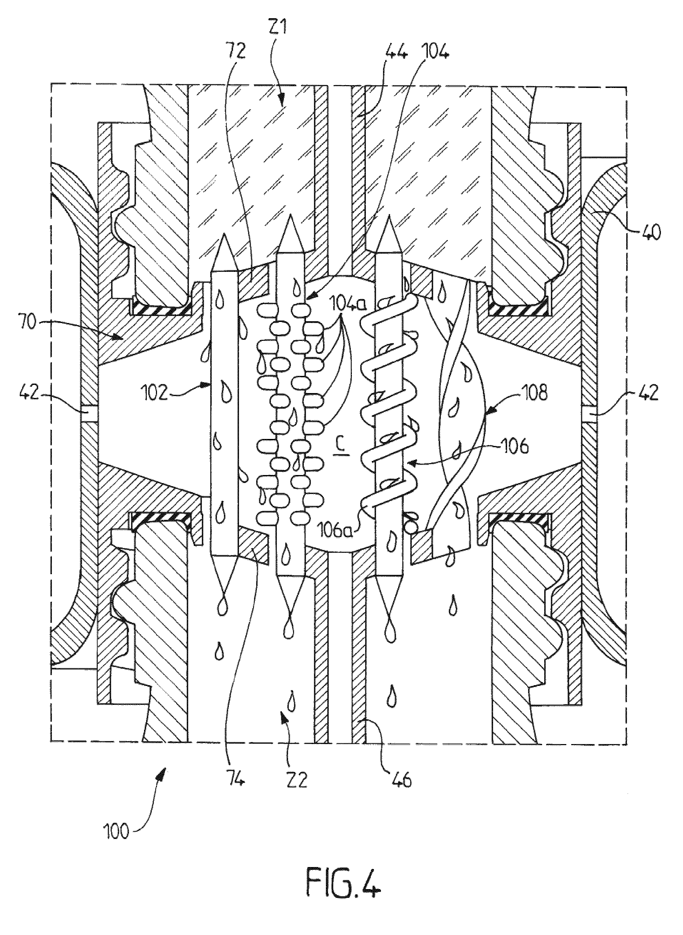

La figure 4 est une vue schématique en coupe axiale d'un dispositif de diffusion de fragrance 100 qui intègre de manière artificielle plusieurs types différents d'obstacles ou de corps de déviation d'écoulement (éléments retardateur d'écoulement) et qui constituent des variantes des tubes de la figure 3.

Pour la commodité de l'exposé, les différents types sont réunis dans la même cavité centrale C mais un dispositif 100 peut très bien ne comporter qu'un seul type d'élément retardateur d'écoulement.

Les composants identiques à ceux de la figure 3 conservent les mêmes références.

Dans cette variante, les éléments ont pour fonction de retenir le plus longtemps possible dans la cavité C (chambre de diffusion) le liquide qui s'écoule du flacon supérieur en déviant sa trajectoire suivant différents chemins simultanés. Les gouttes s'écoulent le long de ces éléments et restent dessus même si le dispositif est retourné (limitation des coulures).

Ces éléments allongés sont montés par leurs extrémités opposées supérieure et inférieure dans les parois en correspondance 72 et 74.

Ces éléments allongés illustrés sur la figure 4 sont :
- un élément 102 en forme de torpille simple (cylindre lisse pointu à ses deux extrémités),
- un élément 104 en forme de torpille et pourvue de picots 104a sur sa surface longitudinale externe,
- un élément 106 en forme de torpille autour de laquelle est monté un ressort en spirale 106a,
- un élément 108 en forme d'hélice.

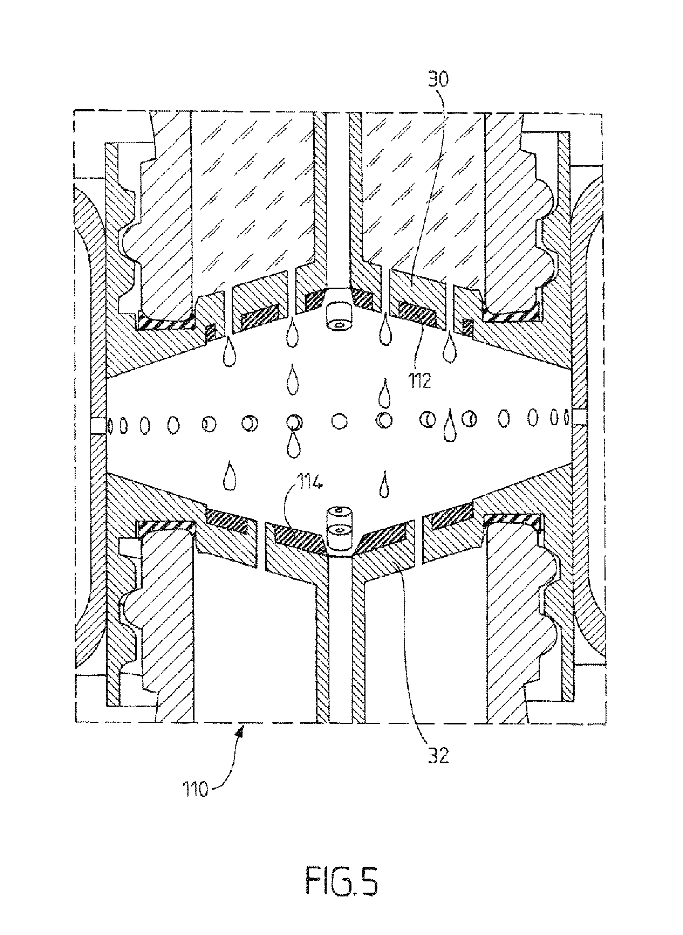

La figure 5 illustre un dispositif 110 selon une variante de réalisation du dispositif de la figure 1. Dans le dispositif 110, des éléments en matière absorbante 112, 114 (ex : pastille en feutre) sont aménagés sur les parois 30, 32 (collecteurs) afin d'y retenir temporairement le liquide (parfum).

Cette matière capte le liquide et reste ainsi humide. Lorsque la matière absorbante 114 de la paroi inférieure est saturée de liquide, l'écoulement naturel de liquide se dirige vers le conduit central 46.

Ainsi, lors du retournement du dispositif en cours d'utilisation, le liquide est retenu par la matière absorbante et n'inonde donc pas la cavité (forte limitation des coulures).

De plus, le liquide (parfum) est retenu plus longtemps dans la cavité par ces éléments en matière absorbante (effet retardateur des éléments), ce qui contribue à améliorer les performances olfactives du dispositif.

On notera que le système représenté à la figure 6 s'applique également aux différents modes de réalisation et variantes décrits en référence aux figures 3 à 5 à la différence près de structure du dispositif de diffusion de fragrance.

## Revendications

1. Dispositif (20 ; 70) de diffusion d'une fragrance, **caractérisé en ce qu'**il comprend :
- deux parois (30, 32 ; 72, 74) en regard espacées l'une de l'autre suivant un axe longitudinal (L) vertical de manière à définir entre elles une cavité (C) qui s'étend transversalement jusqu'à une paroi périphérique (40) pourvue d'au moins une ouverture (42) pour la diffusion de fragrance à l'extérieur du dispositif, ladite au moins une ouverture mettant en communication la cavité avec l'extérieur du dispositif,
- plusieurs ouvertures (O1, 02 ; O'1, O'2) aménagées dans chacune des deux parois en regard et qui traversent la paroi concernée de manière à permettre, pour une première (30) des deux parois située au-dessus de la deuxième paroi (32), le passage de la fragrance liquide d'une première zone (Z1) dans la cavité en vue de la diffusion de ladite fragrance à l'extérieur du dispositif via ladite au moins une ouverture de diffusion, la première zone étant adjacente à ladite première paroi, au-dessus de celle-ci et séparée de la cavité par cette première paroi et, pour la deuxième (32) des deux parois, le passage de la fragrance liquide de la cavité à une deuxième zone (Z2) qui est adjacente à ladite deuxième paroi, en dessous de celle-ci et séparée de la cavité par cette deuxième paroi,
- au moins un conduit (44,46) qui traverse chacune des deux parois en regard et s'étend dans la zone adjacente à la paroi concernée de manière à mettre en communication chaque zone adjacente à chaque paroi avec la cavité, ledit au moins un conduit aménagé dans la première paroi (30) étant un conduit de reprise d'air (44) qui reprend directement de l'air de l'extérieur du dispositif via ladite au moins une ouverture (42) et la cavité, tandis que ledit au moins un conduit (46) aménagé dans la deuxième paroi (32) est un conduit d'écoulement de la fragrance liquide provenant de la cavité.

2. Dispositif selon la revendication 1, **caractérisé en ce que** ledit au moins un conduit de reprise d'air (44) s'étend dans la première zone (Z1) sur une hauteur adaptée pour que l'extrémité débouchante (44a) dudit au moins un conduit de reprise d'air soit à une cote supérieure à celle du niveau de fragrance liquide contenue dans la première zone (Z1).

3. Dispositif selon la revendication 1 ou 2, **caractérisé en ce que** au moins certaines ouvertures aménagées dans les deux parois en regard sont prolongées chacune par un col qui fait saillie dans la cavité par rapport à la paroi concernée.

4. Dispositif selon l'une des revendications 1 à 3, **caractérisé en ce que** les ouvertures (O1, 02 ; O'1, O'2) d'une des deux parois en regard sont décalées géométriquement par rapport aux ouvertures de l'autre paroi.

5. Dispositif selon l'une des revendications 1 à 4, **caractérisé en ce qu'**il comprend plusieurs éléments retardateur d'écoulement de la fragrance liquide (76 ; 102, 104, 106, 108) comprenant chacun un corps de forme générale allongée, les corps s'étendant dans la cavité de manière à relier chacun deux ouvertures respectives des deux parois en regard, permettant ainsi à la fragrance liquide d'être acheminée via chaque élément retardateur d'écoulement d'une ouverture de la première paroi (30) à une ouverture de la deuxième paroi (32).

6. Dispositif selon la revendication 5, **caractérisé en ce que** les corps des éléments retardateur d'écoulement de la fragrance sont formés de plusieurs tubes (76) qui s'étendent dans la cavité de manière à ce que chaque tube relie entre elles deux ouvertures respectives des deux parois en regard, permettant ainsi à la fragrance liquide d'être acheminée via chaque tube d'une ouverture de la première paroi à une ouverture de la deuxième paroi.

7. Dispositif selon l'une des revendications 1 à 6, **caractérisé en ce que** chacune des deux parois en regard (30, 32 ; 72, 74) présente une forme générale concave dont la concavité est orientée vers la cavité et comporte une région centrale.

8. Dispositif selon l'une des revendications 1 à 7, **caractérisé en ce que** ledit au moins un conduit (44,46) est situé dans la région centrale.

9. Dispositif selon l'une des revendications 5 et 6 et selon la revendication 8, **caractérisé en ce que** les éléments retardateur d'écoulement de la fragrance liquide (76; 102, 104, 106, 108) sont disposés autour dudit au moins un conduit.

10. Dispositif selon l'une des revendications 1 à 9, **caractérisé en ce qu'**il comprend un ou plusieurs éléments en matière absorbante (112, 114) aménagés sur chacune des deux parois en regard (30, 32).

11. Système (10 ; 60) de diffusion d'une fragrance, **caractérisé en ce qu'**il comprend :
- un dispositif de diffusion (20 ; 70) selon l'une des revendications 1 à 10,
- un premier flacon supérieur (12) ayant un premier col (12a) dont l'ouverture est disposée en vis-à-vis de la première (30 ; 72) des deux parois en regard dite paroi supérieure, un second flacon inférieur (14) ayant un deuxième col (14a) dont l'ouverture est disposée en vis-à-vis de la deuxième paroi dite inférieure (32 ; 74), le premier flacon supérieur comprenant la fragrance liquide qui, par écoulement sous forme de goutte à goutte à travers les ouvertures (O1, 02 ; O'1, O'2) provoque, d'une part, la diffusion de la fragrance dans la cavité (C) et à l'extérieur du dispositif *via* ladite au moins une ouverture (42) et, d'autre part, le goutte à goutte dans le second flacon inférieur.

12. Procédé de diffusion d'une fragrance dans un dispositif (20 ; 70) de diffusion d'une fragrance, **caractérisé en ce que** le dispositif comprend :
- deux parois (30, 32 ; 72, 74) en regard espacées l'une de l'autre suivant un axe longitudinal (L) vertical de manière à définir entre elles une cavité (C) qui s'étend transversalement jusqu'à une paroi périphérique (40) pourvue d'au moins une ouverture (42) pour la diffusion de fragrance à l'extérieur du dispositif, ladite au moins une ouverture mettant en communication la cavité avec l'extérieur du dispositif,
- plusieurs ouvertures (O1, 02 ; O'1, O'2) aménagées dans chacune des deux parois en regard et qui traversent la paroi concernée de manière à permettre, pour une première (30) des deux parois située au-dessus de la deuxième paroi (32), le passage de la fragrance liquide d'une première zone (Z1) dans la cavité en vue de la diffusion de ladite fragrance à l'extérieur du dispositif via ladite au moins une ouverture de diffusion, la première zone étant adjacente à ladite première paroi, au-dessus de celle-ci et séparée de la cavité par cette première paroi et, pour la deuxième (32) des deux parois, le passage de la fragrance liquide de la cavité à une deuxième zone (Z2) qui est adjacente à ladite deuxième paroi, en dessous de celle-ci et séparée de la cavité par cette deuxième paroi,
- au moins un conduit (44,46) qui traverse chacune des deux parois en regard et s'étend dans la zone adjacente à la paroi concernée de manière à mettre en communication chaque zone adjacente à chaque paroi avec la cavité, ledit au moins un conduit aménagé dans la première paroi (30) étant un conduit de reprise d'air (44), tandis que ledit au moins un conduit (46) aménagé dans la deuxième paroi (32) est un conduit d'écoulement de la fragrance provenant de la cavité, selon le procédé la fragrance liquide contenue dans la première zone (Z1) s'écoule à travers les ouvertures (O1 ; O'1) de la première paroi (30) pour que, d'une part, la fragrance diffuse dans la cavité (C) et à l'extérieur du dispositif via ladite au moins une ouverture (42) et, d'autre part, la partie restante de la fragrance liquide non diffusée s'écoule dans la deuxième zone (Z2) par ledit au moins un conduit (46), l'écoulement de fragrance liquide à travers les ouvertures de la première paroi (30) étant permis par la reprise d'air extérieur via ladite au moins une ouverture (42), la cavité (C) et ledit au moins un conduit de reprise d'air (44).

13. Procédé selon la revendication 12, **caractérisé en ce que** l'écoulement de fragrance liquide depuis la première zone (Z1) à travers les ouvertures (O1 ; O'1) de la première paroi (30) est dévié, lors de son passage dans la cavité, d'un trajet direct pour atteindre la deuxième paroi (32) afin d'augmenter le temps de présence dudit écoulement dans ladite cavité.

14. Procédé selon la revendication 13, **caractérisé en ce que** l'écoulement de fragrance liquide est dévié par sa rencontre avec une pluralité d'obstacles (76 ; 102 ; 104 ; 106 ; 108) présents dans la cavité (C).

15. Procédé selon l'une des revendications 12 à 14, **caractérisé en ce que** la diffusion de la fragrance a lieu dans un système (10 ; 60) de diffusion d'une fragrance, comprenant :
- un dispositif de diffusion de fragrance (20 ; 70),
- un premier flacon supérieur (12) ayant un premier col (12a) dont l'ouverture est disposée en vis-à-vis de la première (30 ; 72) des deux parois en regard dite paroi supérieure, un second flacon inférieur (14) ayant un deuxième col (14a) dont l'ouverture est disposée en vis-à-vis de la deuxième paroi dite inférieure (32 ; 74), le premier flacon supérieur comprenant la fragrance.
